# EUROPÄISCHE PATENTANMELDUNG

(11) **EP 4 137 813 A1**
(43) Veröffentlichungstag der Anmeldung: **22.02.2023**
(21) Anmeldenummer: 22190769.4
(22) Anmeldetag: 17.08.2022
(51) Int. Cl.: G01N 33/02

(54) **SYSTEM UND VERFAHREN ZUR AUTOMATISCHEN QUALITÄTSPRÜFUNG VON OBST UND GEMÜSE UND ANDEREN LEBENSMITTELN**

(30) Priorität: 20.08.2021 DE 102021209199; 13.10.2021 DE 102021211548
(71) Anmelder: Fraunhofer-Gesellschaft zur Förderung der angewandten Forschung e.V., 80686 München (DE); Hochschule für Technik und Wirtschaft des Saarlandes, 66117 Saarbrücken (DE)
(72) Erfinder: Osman, Ahmad, 66123 Saarbrücken (DE); Albert-Weiß, Dominique, 66123 Saarbrücken (DE); Hajdini, Egla, 66111 Saarbrücken (DE)
(74) Vertreter: Pfenning, Meinig & Partner mbB

(57) **Zusammenfassung**

Die Erfindung betrifft ein Verfahren und ein System (10) zur automatischen Qualitätsprüfung von Obst und Gemüse und anderen Lebensmitteln, aufweisend:
- eine erste Messstation (11), die entlang eines Transportpfades des Lebensmittels (16) positionierbar ist und die wenigstens einen Thermografiesensor (12) zum Erfassen von Thermografiedaten (T) des Lebensmittels (16) aufweist;
- eine zweite Messstation (13), die entlang des Transportpfades des Lebensmittels (16) positionierbar ist und wenigstens einen Kamerasensor (26) zum Erfassen von Bilddaten (B) des Lebensmittels (16) auf Basis elektromagnetischer Strahlung im sichtbaren Wellenlängenbereich umfasst;
- eine Ermittlungseinrichtung (15), die dazu eingerichtet ist, auf Basis der Thermografiedaten (T) und der Bilddaten (B) sowie mittels eines Maschinenlernmodellsystems (100) wenigstens eine von einem Reifegrad (R) und einer Haltbarkeit (H) des Lebensmittels (16) zu ermitteln.

## Beschreibung

Eine Herausforderung für die Lebensmittelindustrie besteht darin, die Qualität von Lebensmitteln zu sichern und die Lebensmittelverschwendung zu reduzieren. Es existieren zerstörungsfreie Prüfungsverfahren von Lebensmitteln, die es ermöglichen, Rückschlüsse auf die Qualität der Lebensmittel während ihres gesamten Lebenszyklus zu ziehen, ohne die Lebensmittel zum Beispiel zu zerschneiden oder öffnen zu müssen.

Die Untersuchung der Qualität von Lebensmitteln und insbesondere von landwirtschaftlichen Erzeugnissen kann jedoch aufwendig und/oder ungenau sein, da z.B. ein die Qualität bestimmender Reifeprozess durch die Interaktion vieler biochemischer und biophysikalischer Veränderungen bestimmt wird.

So existieren mechanische Sortieranlagen, die in der Regel in ein Förderband integriert sind und die eine automatische Sortierung der landwirtschaftlichen Erzeugnisse auf Basis erfasster äußerer Eigenschaften wie Gewicht, Größe und Geometrie durchführen. Meist wird zur Erfassung dieser Eigenschaften ein System zum maschinellen Sehen bzw. ein Bildverarbeitungssystem eingesetzt und die Sortierung basiert auf einem Überschreiten oder nicht-Überschreiten vordefinierter Schwellenwerte der vorstehenden Eigenschaften

Ein Beispiel eines existierenden Sortiersystems zum maschinellen Sehen findet sich in der WO 2021/037417 A1. Die EP 0620051 A1 offenbart zudem ein Bildverarbeitungssystem, das mittels eines Beleuchtens von landwirtschaftlichen Produkten bei verschiedenen Wellenlängen kolorimetrische Informationen erzeugt. Diese Systeme können zwar eine gültige Klassifizierung der Lebensmittel in verschiedene Gruppen auf der Grundlage der äußeren Untersuchung ermöglichen, aber sie bewerten die Produkte nicht in Bezug auf andere qualitätsbezogene Merkmale, wie Geschmack oder Haltbarkeit.

Die EP 3589939 A1 offenbart einen Messaufbau mit verschiedenen Lichtquellen, um Lichtstrahlung in verschiedenen Wellenlängenbereichen und gemäß einer vorgegebenen Beleuchtungssequenz auf ein Lebensmittel einzustrahlen. Dabei werden sowohl Bilder im sichtbaren als auch im infraroten Bereich aufgenommen. Das Erzeugen der Beleuchtungssequenzen führt zu einer Verlängerung der Messzeit und zu einer großen Redundanz an Messinformationen, was eine Messauswertung verkompliziert.

Die Erfindung stellt sich demnach die Aufgabe, Qualitätsgrößen von Lebensmitteln zuverlässig und mit begrenztem Aufwand zu ermitteln.

Diese Aufgabe wird durch die Gegenstände der beigefügten unabhängigen Ansprüche gelöst, wobei vorteilhafte Ausführungsformen in den abhängigen Ansprüchen definiert sind.

Insbesondere vorgeschlagen wird ein bevorzugt fest installiertes oder fest installierbares System zur automatischen Qualitätsprüfung von Obst und Gemüse und anderen Lebensmitteln, aufweisend:
- eine erste Messstation, die entlang eines Transportpfades des Lebensmittels positioniert oder positionierbar ist und die wenigstens einen Thermografiesensor zum Erfassen von Thermografiedaten des Lebensmittels aufweist;
- eine zweite Messstation, die entlang eines Transportpfades des Lebensmittels positioniert oder positionierbar ist und die wenigstens einen Kamerasensor zum Erfassen von Bilddaten des Lebensmittels auf Basis elektromagnetischer Strahlung im sichtbaren Wellenlängenbereich umfasst;
- eine Ermittlungseinrichtung, die dazu eingerichtet ist, auf Basis der Thermografiedaten und der Bilddaten sowie mittels eines Maschinenlernmodellsystems (mit wenigstens einem Maschinenlernmodell) einen Reifegrad des Lebensmittels sowie wenigstens eine von einer Haltbarkeit und einem Zuckergehalt zu ermitteln.

Allgemein wird also ein multimodaler oder auch multisensorischer Ansatz vorgeschlagen, bei dem eine visuelle Bildgebung (d. h. eine Bildgebung basierend auf Wellenlängen im sichtbaren Bereich) in Verbindung mit (Infrarot-) Thermografie eingesetzt wird. Hiermit können bevorzugt in Echtzeit jegliche der hierin offenbarten Qualitätsgrößen und speziell der Reifegrad oder die Haltbarkeit bestimmt werden, was auch ein Erfassen innerer und/oder äußerer Defekte (insbesondere Oberflächendefekte) umfassen kann.

Durch die Anreicherung der erfassten Messinformationen mit Thermografiedaten (insbesondere in Form von Wärmebildern) können insbesondere physiologische Störungen oder Defekte erkannt werden, die mit existierenden Systemen des maschinellen Sehens nicht berücksichtigt werden. Weiter ermöglicht insbesondere der Einsatz eines Maschinenlernmodells eine verbesserte Inferenz- und Verallgemeinerungsfähigkeit sowie das Ermitteln bisher nicht erfasster Qualitätsgrößen, wie beispielsweise einer erwarteten Haltbarkeit oder geschmacklicher Größen basierend auf dem Zuckergehalt.

Die Vorhersage der Haltbarkeit ermöglicht eine bessere Planung innerhalb der Lieferkette. Beispielsweise kann dies bei einem Versand von insbesondere klimakterischen Früchten eine Ausfallwahrscheinlichkeit einer Charge reduzieren, bei der andernfalls reife und/oder beschädigte Früchte ein Verderben der gesamten Charge verursachen könnten.

Schließlich ermöglicht die Lösung einen wenig komplexen Aufbau sowie eine kurze Messzeit, was eine Integration und insbesondere feste Installation in automatischen Sortier- und Verpackungsanlagen vereinfacht.

Das System kann fest in einer Sortier-, Verpackungs- oder einer allgemeinen industriellen Verarbeitungsanlage der Lebensmittel installiert sein. Es kann sich demnach um ein festinstalliertes System handeln, das zum Beispiel nicht für flexible und/oder lediglich temporäre Einsätze zum Beispiel außerhalb der genannten Anlagen vorgesehen ist. Weiter ist das System bevorzugt für einen automatischen Betrieb eingerichtet und kein manuell bedienbares System für eine manuell gesteuerte Lebensmittelprüfung. Beispielsweise weist das System bevorzugt keine handhaltbaren und/oder manuell beweglichen Teile auf, insbesondere keine handhaltbaren und/oder manuell beweglichen Teile, die Sensordaten der hierin offenbarten Art erfassen können.

Die Messstationen sind bevorzugt räumlich getrennt und beispielsweise entlang eines Transportpfades der Lebensmittel voneinander beanstandet. Prinzipiell ist es aber auch möglich, dass die Messstationen räumlich überlappend angeordnet sind und/oder in einen gemeinsam räumlichen Bereich zum Beispiel entlang eines Förderbands integriert sind. Die genannte Überlappung oder Integration kann beispielsweise umfassen, dass sich die Erfassungsbereiche der jeweiligen Sensoren entsprechend überlappen und/oder zusammenfallen.

Der Thermografiesensor kann eine Wärmebildkamera sein. Allgemein kann der Thermografiesensor dazu eingerichtet sein, als Thermografiedaten eine Strahlungsintensität von Infrarotstrahlung zu erfassen, die beispielsweise von dem zu überprüfenden Lebensmittel ausgeht. Die Thermografiedaten können in Form wenigstens eines Wärmebildes gespeichert werden.

Bevorzugt ist der Thermografiesensor ein aktiver Thermografiesensor, der eine Wärmequelle zum Beispiel in Form einer wärmestrahlenden Lampe umfasst (zum Beispiel eine Blitzlampe). In an sich bekannter Weise kann hierüber ein Wärmefluss und/oder eine Wärmeleitung einer mittels der Wärmequelle in das zu untersuchende Lebensmittel eingebrachten Wärme erfasst werden, indem wiederum Thermografiedaten der oben genannten Art ermittelt werden. Beispielsweise können anhand von Inhomogenitäten des Wärmeflusses Defekte, insbesondere innere Defekte, des Lebensmittels erkannt werden.

Die Bilddaten umfassen bevorzugt unterschiedliche Farbkanäle, insbesondere Pixelwerte für unterschiedliche Farbkanäle. Bevorzugt werden RGB-Farbkanäle erfasst (rot, grün, blau).

Bei der Ermittlungseinrichtung kann es sich um eine computerbasierte Steuereinrichtung handeln, die beispielsweise wenigstens einen Prozessor umfasst. Der Prozessor ist bevorzugt dazu eingerichtet, die erhaltenen Sensordaten (also die Thermografiedaten, die Bilddaten und jegliche anderen hierin erläuterten und an die Ermittlungseinrichtung übermittelten sensorischen Daten) gemäß jeglichem der hierin geschilderten Ansätze zu verarbeiten, insbesondere unter Verwendung des Maschinenlernmodellsystems. Beispielsweise kann die Ermittlungseinrichtung dazu eingerichtet sein, ein Computerprogramm und/oder Computeralgorithmen auszuführen, die das Maschinenlernmodellsystem implementieren.

Das Maschinenlernmodellsystem kann wenigstens ein künstliches neuronales Netz als Beispiel eines einzelnen Maschinenlernmodells umfassen. Allgemein kann es sich um einen Algorithmus und/oder zumindest einen Teil eines Computerprogramms handeln, der Verarbeitungsvorschriften definiert, um den Reifezustand und/oder die Haltbarkeit zu ermitteln. Insbesondere kann das Maschinenlernmodellsystem Zusammenhänge zwischen den Sensordaten (d.h. der Bild- und Thermografiedaten) und/oder darauf basierend erzeugter Eingangsdaten des Maschinenlernmodellsystems und dem Reifezustand und/oder der Haltbarkeit definieren. Diese Zusammenhänge können im Rahmen eines maschinellen Lernvorgangs erlernt werden. Dieser Lernvorgang (auch als Training bezeichnet) kann ein sogenannter supervised-Lernvorgang unter Verwendung von Trainingsdatensätzen sein, die verifizierte Zusammenhänge aus Sensordaten und dabei vorliegenden Qualitätsgrößen in Form des Reifzustandes und/oder der Haltbarkeit enthalten.

Der Reifegrad kann in definierte Grade unterteilt sein, zum Beispiel in Untergruppen von reif, unreif, überreif. Die Haltbarkeit kann in einer definierten Zeiteinheit oder als ein maximales Datum angegeben werden, ab dem eine Haltbarkeit (bzw. Genießbarkeit) des Lebensmittels als nicht mehr gegeben angenommen wird.

Der Reifegrad und die Haltbarkeit können Beispiele von Ermittlungsergebnissen und/oder Qualitätsgrößen sein, die das Maschinenlernmodellsystem ermittelt. Sie können zum Beispiel mittels einer optionalen Anzeigeeinrichtung des Systems einem Benutzer angezeigt werden, beispielweise einem Benutzer, der eine das System umfassende Sortieranlage steuert und überwacht. Alternativ oder zusätzlich können die Ermittlungsergebnisse zum Erzeugen der nachstehenden Steuersignale für eine Sortiereinrichtung verwendet werden.

Die erste Messstation umfasst bevorzugt einen Bewegungsaktor zum Erzeugen einer Relativbewegung zwischen dem Thermografiesensor und dem Lebensmittel. Auf diese Weise kann das Lebensmittel von verschiedenen Seiten erfasst werden, beispielsweise von wenigstens zwei oder wenigstens vier Seiten. Gemäß einer Variante ist der Bewegungsaktor dazu eingerichtet, das Lebensmittel (bevorzugt aktiv) relativ zu dem (bevorzugt stillstehenden) Thermografiesensor zu rotieren. In jeder von einer Mehrzahl definierter Rotationsstellungen des Bewegungsaktors erfasst der Thermografiesensor vorzugsweise Thermografiedaten des Lebensmittels.

Die erste Messstation kann auch den nachstehend erläuterten Umgebungssensor umfassen.

Die zweite Messstation umfasst bevorzugt eine Abschirmungseinrichtung zum zumindest teilweisen Abschirmen des Lebensmittels gegenüber Umgebungslicht während einer Erfassung durch den Kamerasensor. Beispielsweise kann es sich um eine lichtundurchlässige Verkleidung handeln (genauer gesagt: undurchlässig für Licht im sichtbaren Wellenlängenbereich). Die Verkleidung kann einen Auflagebereich für das Lebensmittel, in dem das Lebensmittel für ein Erfassen durch den Kamerasensor positioniert ist, zumindest teilweise umgeben. Der Erfassungsbereich des Kamerasensors kann den Auflagebereich einschließen und/oder zumindest teilweise innerhalb der Verkleidung liegen. Allgemein kann die Verkleidung Öffnungen für ein Einführen und/oder Ausführen des Lebensmittels sowie, zusätzlich oder alternativ, für den Kamerasensor umfassen, damit dieser das Lebensmittel erfassen kann.

Allgemein kann die Verkleidung das Vorliegen definierter und bevorzugt konstanter Lichtbedingungen fördern, was für eine Auswertung der Bilddaten vorteilhaft ist.

Die erste Messstation kann optional ebenfalls einen Bewegungsaktor der vorstehend geschilderten Art umfassen. Dieser kann dazu eingerichtet sein, eine Relativbewegung zwischen dem Thermografiesensor und dem Lebensmittel zu erzeugen insbesondere Letzteres von verschiedenen Seiten zu erfassen. Gemäß einer Variante ist der Bewegungsaktor dazu eingerichtet, das Lebensmittel (bevorzugt aktiv) relativ zu dem (bevorzugt stillstehenden) Thermografiesensor zu rotieren. In jeder aus einer Mehrzahl definierter Rotationsstellungen des Bewegungsaktors erfasst der Kamerasensor vorzugsweise Thermografiedaten des Lebensmittels.

Alternativ oder zusätzlich zu den erwähnten optionalen Bewegungsaktoren der ersten und zweiten Messstation können diese Messstationen jeweils eine Mehrzahl von Thermografiesensoren bzw. Kamerasensoren umfassen. Diese können derart angeordnet sein, dass sie bevorzugt ohne das Erzeugen einer zusätzlichen Relativbewegung (d. h. zusätzlich zu einer Transportbewegung des Lebensmittels in die und aus der Messstation) zwischen dem Lebensmittel und dem Sensor das Lebensmittel von verschiedenen Seiten erfassen können.

Die Bilddaten und/oder die Thermografiedaten werden bevorzugt vorverarbeitet, bevor sie als Eingangsgrößen dem Maschinenlernmodellsystem zugeführt werden oder bevor derartige Eingangsgrößen aus den Bilddaten und/oder Thermografiedaten ermittelt werden.

Im Fall der Bilddaten kann die Vorverarbeitung wenigstens eine der folgenden Maßnahmen umfassen:
- ein Beschneiden der Bilddaten (insbesondere eines jeweiligen hiervon definierten Bildes) auf einen definierten Interessensbereich (engl.: Region of Interest). Hierfür kann zunächst eine Segmentierung des Bildes und/oder Identifizierung von wenigstens einem das Lebensmittel enthaltenen Bildsegments erfolgen. Diese Segmentierung und/oder allgemein das Beschneiden kann mittels des Maschinenlernmodells erfolgen werden.
- Die Bildgröße kann, insbesondere nachfolgend auf ein Beschneiden, mittels einer bevorzugt bikubischen Interpolation neu berechnet werden, was die benötigte Zeit für eine Auswertung durch das Maschinenlernmodellsystem reduziert.
- Die einzelnen Farbkanäle (insbesondere RGB-Farbkanäle) können normalisiert werden, was eine gleichartige Auswertung der einzelnen Kanäle durch das Maschinenlernmodellsystem verbessert.

Im Fall der Thermografiedaten kann die Vorverarbeitung wenigstens eine der folgenden Maßnahmen umfassen:
- Die Thermografiedaten können frequenztransformiert werden (genauer gesagt von dem Zeitbereich in den Frequenzbereich transformiert werden), vorzugsweise mittels einer eindimensionalen diskreten Fouriertransformation (DFT). Dies kann das Ausmaß redundanter Informationen innerhalb der Bilddaten reduzieren, die beispielsweise durch Absorptionen oder Reflexionen hervorgerufen werden.
- Die Thermografiedaten, insbesondere ein hiervon definiertes und optional frequenztransformiertes Wärmebild, können per Kontrastspreizung (engl.: contrast stretching) bearbeitet werden. Anschließend kann eine Normalisierung und/oder Neuberechnung der Größe des Wärmebildes erfolgen.

Gemäß einer Weiterbildung sind die erste und zweite Messstation räumlich getrennt (d.h. in einem Abstand zueinander positioniert) und vorzugsweise durch eine Transporteinrichtung (z.B. ein Förderband) für den automatischen Transport des Lebensmittels zwischen den Messstationen verbunden.

Das System umfasst gemäß einer Ausführungsform auch eine Sortiereinrichtung, die dazu eingerichtet ist, die Lebensmittel auf Basis des Ermittlungsergebnisses der Ermittlungseinrichtung automatisch zu sortieren, insbesondere aus einem Haupt-Transportpfad auszusortieren. Beispielsweise kann die Ermittlungseinrichtung Steuersignale oder Steueranweisungen erzeugen, die die Sortiereinrichtung veranlassen, eine geeignete Sortierung und insbesondere eine selektive Aussortierung vorzunehmen. Die Sortiereinrichtung kann wenigstens einen Aktor aufweisen, mit dem das zum Sortieren Lebensmittel bewegbar ist, beispielsweise durch Entfernen von einem Förderband. Alternativ oder zusätzlich kann eine Sortierung zumindest zunächst dadurch erfolgen, dass die Sortiereinrichtung das Lebensmittel geeignet markiert (beispielsweise durch einen computerlesbaren Marker, wie einen Barcode oder einer an dem Produkt oder einer mit dem Produkt evrsehenen Palette angebrachten RFID-Tag (radio frequency identification)). Mittels einer solchen Markierung können die Lebensmittel in unterschiedliche Klassen eingeteilt und somit sortiert werden. So kann der RFID-Tag beispielsweise Informationen über die vorhergesagte Produktkategorie und zugleich eine zusätzliche Information wie zum Beispiel den Zuckergehalt (Brix-Wert) bei Früchten oder den Fettanteil in Fisch oder Fleisch enthalten.

Gemäß einem optionalen Aspekt umfasst das System wenigstens einen Umgebungssensor zum Erfassen wenigstens einer Umgebungsgröße, vorzugsweise der Temperatur oder der Luftfeuchtigkeit, wobei die Ermittlungseinrichtung dazu eingerichtet ist, die Umgebungsgröße als eine Hilfsvariable oder "auxiliary variable" für das Maschinenlernmodellsystem zu verwenden. Diese Hilfsvariable wird typischerweise nach der Komprimierung der Information der akustischen Signale verwendet, in der Regel entweder nach einer "Global Average Pooling" oder "Flattening Layer". Die Verknüpfung der Hilfsvariablen mit den extrahierten Informationen der akustischen Signale erfolgt bei uns durch eine Konkatinierung, kann aber auch durch andere Operationen wie "Add", "Merge" oder "Dot" erfolgen.

Der Umgebungssensor kann alternativ als atmosphärischer Sensor und die Umgebungsgröße kann alternativ als atmosphärische Größe bezeichnet werden. Der Umgebungssensor kann ein Thermo-Hygrometer sein.

Gemäß einer Ausführungsform erfasst ein Erfassungsbereich des Kamerasensors das Lebensmittel zumindest teilweise von oberhalb oder, mit anderen Worten, ist der Erfassungsbereich für eine solche Erfassung von oberhalb ausgerichtet. Der Begriff "oberhalb" bezieht sich dabei vorzugsweise auf eine vertikale Raumachse entlang derer die Gravitationskraft wirkt. Das Lebensmittel kann oder die Lebensmittel können zum Beispiel in einem Auflagebereich für eine Erfassung positioniert sein, während der Kamerasensor oberhalb des Auflagebereichs angeordnet und der Erfassungsbereich auf den Auflagebereich gerichtet ist. Es hat sich gezeigt, dass ein oberer Bereich besonders bei Obst- und Gemüse, Bildinformationen liefert, mit denen zuverlässig auf den Reifegrad und/oder die Haltbarkeit geschlossen werden kann.

Insbesondere kann dieser Bereich eine sogenannte Abzissionszone und/oder den Bereich eines Stils umfassen, mittels der/dem das Obst oder Gemüse mit der tragenden Pflanze verbunden ist bzw. war. In diesem Bereich sind insbesondere visuelle Merkmale erfassbar, die den obigen Rückschluss zuverlässig ermöglichen.

Bevorzugt ist die Ermittlungseinrichtung ferner dazu eingerichtet, mittels des Maschinenlernmodellsystems einen Zuckergehalt des Lebensmittels zu ermitteln. Es hat sich gezeigt, dass insbesondere auf Basis der Thermografiedaten von Obst und Gemüse diese Eigenschaft aussagekräftig ermittelbar ist. Über den Zuckergehalt kann zumindest mittelbar auf den Geschmack oder das Aroma des Lebensmittels geschlossen werden und können darauf basierend Sortierungen vorgenommen werden. Dies ist mit bisherigen und rein auf visuellen Eigenschaften basierenden Sortierungen nicht möglich.

Gemäß einer bevorzugten weiteren Ausführungsform umfasst das Maschinenlernmodellsystem wenigstens ein Convolutional Neural Network (kurz: CNN, deutsch: faltendes neuronales Netzwerk), welches sowohl den Reifegrad als auch die Haltbarkeit ermittelt. Aufgrund des Ermittelns dieser beiden Eigenschaften kann auch von einem Multi-Task CNN gesprochen werden. Bevorzugt erhält dieses als Bild-Eingangsdaten die Bilddaten des Kamerasensors als auch die Thermografiedaten, die einem Wärmebild entsprechen können. Das CNN kann gemäß einem sogenannten hard-parameter-sharing Ansatz trainiert sein, was in einem Teilen von wenigstens einer verborgenen Schicht (engl.: hidden layer) durch die Tasks resultieren kann. Anders ausgedrückt können die Bild-Eingangsdaten zunächst wenigstens einer gemeinsamen Schicht des CNN zugeführt werden, auf die vorzugweise jeweils wenigstens eine taskspezifische Schicht folgen kann. Ein erstes Task (bzw. eine erste Aufgabe) kann das Ermitteln des Reifegrads betreffen, ein zweites Task ein Ermitteln der Haltbarkeit.

Mittels des hard-parameter-sharing Ansatzes kann das Risiko einer Überbestimmung (engl.: overfitting) reduziert werden.

Gemäß einer weiteren Variante ist das Maschinenlernmodellsystem dazu eingerichtet, zum Ermitteln des Reifegrads einen Klassifikationsvorgang durchzuführen und/oder zum Ermitteln der Haltbarkeit einen Regressionsvorgang durchzuführen. Insbesondere können die jeweiligen Ermittlungstasks von einem Multi-Task-Maschinenlernmodell (zum Beispiel in Form eines CNN der obigen Art) diese Vorgänge ausführen, wobei die Tasks in dem Ermitteln des Reifegrads und dem Ermitteln der Haltbarkeit bestehen.

Ferner offenbart wird ein Verfahren zur Qualitätsprüfung von Obst und Gemüse und anderen Lebensmitteln, wobei das Verfahren mittels einem System gemäß einem der vorangehenden Aspekte ausgeführt wird und umfasst:
- Erfassen von Thermografiedaten des Lebensmittels mittels der ersten Messstation;
- Erfassen von Bilddaten des Lebensmittels auf Basis elektromagnetischer Strahlung im sichtbaren Wellenlängenbereich mittels der zweiten Messstation;
- Ermitteln auf Basis der Thermografiedaten und der Bilddaten sowie mittels eines Maschinenlernmodellsystems wenigstens eine von einem Reifegrad und einer Haltbarkeit des Lebensmittels.

Das Verfahren kann jeglichen weiteren Schritt umfassen, um sämtliche in dem Zusammenhang mit dem System geschilderten Betriebszustände und/oder Funktionen bereitzustellen. Beispielsweise kann ein Schritt des Ausführens des Maschinenlernmodellsystems mittels eines Prozessors der Ermittlungseinrichtung und/oder ein Schritt eines bevorzugt automatischen Transportierens des Lebensmittels zwischen den Messstationen vorgesehen sein.

Bevorzugt umfasst das Maschinenlernmodellsystem wenigstens ein Convolutional Neural Network der vorstehend geschilderten Art, welches sowohl den Reifegrad als auch die Haltbarkeit ermittelt, und das Verfahren ist ferner gekennzeichnet durch:
- Trainieren des Convolutional Neural Network auf Basis eines hard-parameter-sharing Ansatzes.

Das Verfahren ist typischerweise mit dem beschriebenen System durchführbar bzw. das beschriebene System ist zum Durchführen des beschriebenen Verfahrens eingerichtet.

Die Erfindung betrifft vorzugsweise auch ein Computerprogrammprodukt mit einem Computerprogramm, das Softwaremittel zum Durchführen des beschriebenen Verfahrens oder zum Steuern des beschriebenen Systems aufweist, wenn das Computerprogramm in einem Automatisierungssystem bzw. durch eine Recheneinheit ausgeführt wird.

Ausführungsbeispiele der Erfindung werden im Folgenden anhand der beigefügten schematischen Figuren erläutert. Figurenübergreifend können für gleichartige oder gleichwirkende Merkmale die gleichen Bezugszeichen verwendet werden.
Fig. 1 zeigt den Aufbau eines Systems gemäß einem Ausführungsbeispiel der Erfindung, wobei das System ein Verfahren gemäß einem Ausführungsbeispiel der Erfindung ausführt;
Fig. 2 zeigt eine Prinzipdarstellung eines Maschinenlernmodellsystems, das von dem System aus Figur 1 ausgeführt wird.

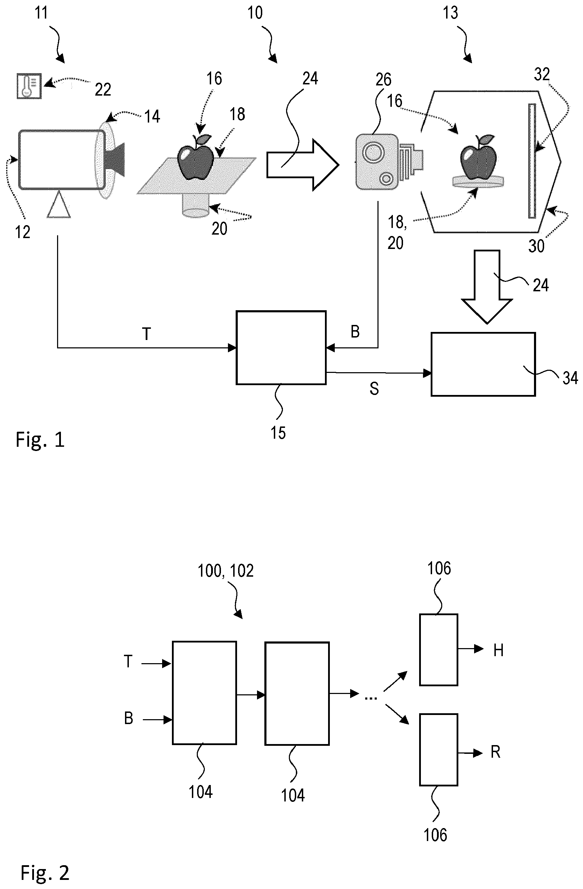

Figur 1 zeigt ein System 10 gemäß einem Ausführungsbeispiel, wobei das System 10 für eine Qualitätsüberprüfung und Sortierung von Lebensmitteln und im gezeigten Beispiel von Obst oder Gemüse eingerichtet ist. Das System 10 ist fest in zum Beispiel einer Fabrik- oder Produktionsumgebung installiert.

Das System 10 umfasst eine erste Messstation 11 mit einem Thermografiesensor 12 in Form einer Wärmebildkamera. Ferner umfasst es eine zweite Messstation 13 mit einem Kamerasensor 26 für Erfassungen des Lebensmittels 16 im sichtbaren Wellenlängenbereich.

Die erste und zweite Messstation 11, 13 sind über eine Transporteinrichtung 24 in Form eines automatischen Förderbandes miteinander verbunden, wobei die Transporteinrichtung 24 lediglich schematisch durch einen Blockpfeil dargestellt ist. Das System 10 umfasst auch eine Sortiereinrichtung 34, zu der das Lebensmittel 16 nach Durchlaufen der beiden Messstationen 11, 13 mittels einer gleichartigen Transporteinrichtung 24 transportierbar ist. Ein Transportpfad des Lebensmittels 16 verläuft entsprechend durch die Messstationen 11, 13 zu der Sortiereinrichtung 34.

Schließlich umfasst das System 10 auch eine Ermittlungseinrichtung 15, die Sensordaten des Thermografiesensors 12 in Form von Thermografiedaten T und Bilddaten B des Kamerasensors 26 erhält. Die Ermittlungseinrichtung 15 gibt Steuerdaten S an die Sortiereinrichtung 34 aus.

Das Lebensmittel 16 gelangt automatisch in die erste Messstation 11 mittels einer nicht gesondert dargestellten Transporteinrichtung, die analog zu den Transporteinrichtung 24 aus Figur 1 ausgebildet sein kann. Das Lebensmittel 16 ist lediglich beispielhaft auf einem Auflagebereich 18 positioniert, der mittels eines Bewegungsaktors 20 relativ zu dem feststehenden Thermografiesensor 12 drehbar ist. In definierten Rotationsstellungen (beispielsweise nach Rotationen um jeweils 90°) erfasst der Thermografiesensor 12 Thermografiedaten T des Lebensmittels 16 und übermittelt diese an die Ermittlungseinrichtung 15.

Dabei ist der Thermografiesensor 12 im gezeigten Beispiel ein aktiver Thermografiesensor 12, der mittels einer Blitzlampe 14 zunächst einen Wärmeeintrag in das Lebensmittel 16 erzeugt und anschließend den Wärmefluss im Lebensmittel 16 erfasst.

Optional umfasst die erste Messstation 11 auch einen Umgebungssensor 22 in Form eines Thermo-Hygrometers. Hiervon erfasste Umgebungsdaten (die Temperatur und die Luftfeuchtigkeit) können ebenfalls an die Ermittlungseinrichtung 15 übermittelt und dort als Hilfsvariablen eines von der Ermittlungseinrichtung 15 ausgeführten Maschinenlernmodells 102 verwendet werden.

In der zweiten Messstation 13 wird das Lebensmittel 16 lediglich beispielhaft frontal oder, mit anderen Worten, in einer Seitenansicht von dem Kamerasensor 26 erfasst. Erneut kann das Lebensmittel 16 dabei in einem Auflagebereich 18 positioniert sein, der mittels eines Bewegungsaktors 20 relativ zu dem bevorzugt feststehenden Kamerasensor 26 bewegbar ist.

Gemäß einer bevorzugten nicht dargestellten Alternative erfasst der Kamerasensor 26 das Lebensmittel 16 von oberhalb, beispielsweise sodass eine optische Achse hiervon gemäß dem Pfeil 16 in Figur 1 auf das Lebensmittel 16 ausgerichtet ist. Wie im allgemeinen Beschreibungsteil erläutert, können hiermit insbesondere für Obst und Gemüse besonders aussagekräftige Bildinformationen erfasst werden.

Die zweite Messstation 13 umfasst bevorzugt auch eine Verkleidung 30, um das Lebensmittel 16 bzw. den Auflagebereich 18 zumindest teilweise gegenüber Umgebungslicht abzuschirmen. Nicht gesondert dargestellt sind Zugangsöffnungen für ein Einführen und Ausführen des Lebensmittels 16 in und aus der Verkleidung 30. Innerhalb der Verkleidung 30 ist vorzugsweise auch ein gegenüberliegend zum Kamerasensor positioniertes Hintergrundelement 32 positioniert, beispielsweise eine Hintergrundplatte. Diese verbessert eine Gleichmäßigkeit der Aufnahmebedingungen und somit beispielsweise eine von der Ermittlungseinrichtung 15 durchgeführte Segmentierung der Bilddaten. Diese Segmentierung kann ein Identifizieren und/oder Herausfiltern von Bildpixeln umfassen, die das Lebensmittel 16 abbilden, und kann mittels eines Maschinenlernmodells erfolgen.

Die Sortiereinrichtung 34 kann jegliche in dem allgemeinen Beschreibungsteil erläuterten Sortiervarianten bereitstellen. Für das zuvor in den Messstationen 11, 13 erfasste Lebensmittel 16 kann die Ermittlungseinrichtung 15 auf Basis der ermittelten Qualitätsgrößen der Haltbarkeit H und des Reifegrads S entscheiden, ob das Lebensmittel 16 von der Sortiereinrichtung 34 aussortiert werden soll oder nicht, und entsprechend erzeugte Steuersignale S an die Sortiereinrichtung 34 ausgeben.

Fig. 2 zeigt ein Beispiel eines Maschinenlernmodellsystems 100, das von der Ermittlungseinrichtung 15 und genauer gesagt einer nicht gesondert dargestellten Prozessoreinrichtung hiervon ausgeführt wird. Das Maschinenlernsystem 100 umfasst im gezeigten Beispiel ein einzelnes Maschinenlernmodell in Form eines CNN 102. Es können jedoch auch mehrere Maschinenlernmodellsysteme, insbesondere für eine Vorverarbeitung der Bilddaten B und/oder Thermografiedaten T vorgesehen sein.

Das CNN 102 ist ein Multi-Task-CNN 102, dass für die Aufgaben bzw. Tasks des Ermittelns eines Reifegrads R und der Haltbarkeit H des Lebensmittels 16 trainiert ist. Das Training erfolgt überwacht oder, mit anderen Worten, als ein überwachter Lernvorgang. Dabei werden verifizierte Datensätze verwendet, die Thermografiedaten T, Bilddaten B sowie dabei jeweils vorliegende Haltbarkeiten H und Reifegrade R eines zu prüfenden Lebensmittels 16 umfassen.

Das CNN 102 umfasst eine Mehrzahl von geteilten verborgenen Schichten 104, deren genaue Zahl beliebig ist (angedeutet durch Punkte in Figur 2). Nicht gesondert dargestellt sind die bei CNN's 102 vorgesehenen spezifischen Pooling- und Faltungsschichten, die jeweils ein Beispiel einer geteilten Schicht 104 sein können. Für jede Aufgabe existiert abschließend zumindest eine gesonderte Schicht 106, die einen eindeutigen Zusammenhang zu den ermittelnden Qualitätsgrößen des Reifegrads R und der Haltbarkeit H definiert.

Wenn diese Qualitätsgrößen, für sich genommen oder in Kombination, ein vorbestimmtes Aussortierkriterium erfüllen, erzeugt die Ermittlungseinrichtung 15 entsprechende Steuersignale S für ein Aussortieren des Lebensmittels 16 durch die Sortiereinrichtung 34.

Nicht gesondert dargestellt ist, dass die Thermografiedaten T und Bilddaten B bevorzugt in der Ermittlungseinrichtung 15 auch gemäß jeglichen hierin geschilderten Varianten vorverarbeitet werden können.

## Patentansprüche

1. System (10) zur automatischen Qualitätsprüfung von Obst und Gemüse und anderen Lebensmitteln, aufweisend:
- eine erste Messstation (11), die entlang eines Transportpfades des Lebensmittels (16) positionierbar ist und die wenigstens einen Thermografiesensor (12) zum Erfassen von Thermografiedaten (T) des Lebensmittels (16) aufweist;
- eine zweite Messstation (13), die entlang des Transportpfades des Lebensmittels (16) positionierbar ist und wenigstens einen Kamerasensor (26) zum Erfassen von Bilddaten (B) des Lebensmittels (16) auf Basis elektromagnetischer Strahlung im sichtbaren Wellenlängenbereich umfasst;
- eine Ermittlungseinrichtung (15), die dazu eingerichtet ist, auf Basis der Thermografiedaten (T) und der Bilddaten (B) sowie mittels eines Maschinenlernmodellsystems (100) wenigstens eine von einem Reifegrad (R) und einer Haltbarkeit (H) des Lebensmittels (16) zu ermitteln.

2. System (10) nach Anspruch 1,
**dadurch gekennzeichnet, dass** die erste und zweite Messstation (11, 13) räumlich getrennt und vorzugsweise durch eine Transporteinrichtung (24) für den automatischen Transport des Lebensmittels (16) zwischen den Messstationen (11, 13) verbunden sind.

3. System (10) nach Anspruch 1 oder 2,
ferner **gekennzeichnet durch** eine Sortiereinrichtung (34), die dazu eingerichtet ist, das Lebensmittel (16) auf Basis des Ermittlungsergebnisses der Ermittlungseinrichtung (15) automatisch zu sortieren.

4. System (10) nach einem der vorangehenden Ansprüche,
ferner **gekennzeichnet durch** wenigstens einen Umgebungssensor (22) zum Erfassen wenigstens einer Umgebungsgröße, vorzugsweise der Temperatur oder der Luftfeuchtigkeit,
wobei die Ermittlungseinrichtung (15) dazu eingerichtet ist, die Umgebungsgröße als eine Hilfsvariable für das Maschinenlernmodellsystem (100) zu verwenden.

5. System (10) nach einem der vorangehenden Ansprüche,
**dadurch gekennzeichnet, dass** ein Erfassungsbereich des Kamerasensors (26) derart ausgerichtet ist, dass das Lebensmittel (16) zumindest teilweise von oberhalb erfassbar ist.

6. System (10) nach einem der vorangehenden Ansprüche,
**dadurch gekennzeichnet, dass** die Ermittlungseinrichtung (15) ferner dazu eingerichtet ist, auf Basis der Thermografiedaten (T) und der Bilddaten (B) sowie mittels des Maschinenlernmodellssystems (100) einen Zuckergehalt des Lebensmittels (16) zu ermitteln.

7. System (10) nach einem der vorangehenden Ansprüche,
**dadurch gekennzeichnet, dass** das Maschinenlernmodellsystem (100) wenigstens ein Convolutional Neural Network (102) umfasst, mit dem sowohl den Reifegrad als auch die Haltbarkeit ermittelbar ist.

8. System (10) nach einem der vorangehenden Ansprüche,
**dadurch gekennzeichnet, dass** das Maschinenlernmodellssystem (100) dazu eingerichtet ist, zum Ermitteln des Reifegrads (R) einen Klassifikationsvorgang durchzuführen und/oder zum Ermitteln der Haltbarkeit (H) einen Regressionsvorgang durchzuführen.

9. Verfahren zur Qualitätsprüfung von Obst und Gemüse und anderen Lebensmitteln, wobei das Verfahren mittels eines Systems (10) gemäß den Ansprüchen 1-8 ausgeführt wird und umfasst:
- Erfassen von Thermografiedaten (T) des Lebensmittels (16) mittels der ersten Messstation (11);
- Erfassen von Bilddaten (B) des Lebensmittels (16) auf Basis elektromagnetischer Strahlung im sichtbaren Wellenlängenbereich mittels der zweiten Messstation (13);
- Ermitteln auf Basis der Thermografiedaten (T) und der Bilddaten (B) sowie mittels eines Maschinenlernmodellsystems (100) wenigstens eine von einem Reifegrad (R) und einer Haltbarkeit (H) des Lebensmittels (16).

10. Verfahren nach Anspruch 9,
wobei das Maschinenlernmodellsystem (100) wenigstens ein Convolutional Neural Network (102) umfasst, welches sowohl den Reifegrad (R) als auch die Haltbarkeit (H) ermittelt, und das Verfahren ferner **gekennzeichnet ist durch**:
- Trainieren des Convolutional Neural Network (102) auf Basis eines hard-parameter-sharing Ansatzes.
